# EUROPÄISCHE PATENTANMELDUNG

(11) **EP 3 406 593 A1**
(43) Veröffentlichungstag der Anmeldung: **28.11.2018**
(21) Anmeldenummer: 17172737.3
(22) Anmeldetag: 24.05.2017
(51) Int. Cl.: C07C 319/28, C25B 3/00, C07C 323/58

(54) **VERFAHREN ZUR HERSTELLUNG VON METHIONIN**

(71) Anmelder: Evonik Degussa GmbH, 45128 Essen (DE)
(72) Erfinder: FISCHER, Daniel, Midlothian, VA 23113 (US); STENNER, Patrik, 63452 Hanau (DE); BERNHARDT, Sebastian, 63739 Aschaffenburg (DE); JAKOB, Harald, 63594 Hasselroth (DE)

(57) **Zusammenfassung**

Die vorliegende Erfindung betrifft ein Verfahren zur Herstellung von Methionin, umfassend die Hydrolyse von 5-(2-Methylmercaptoethyl)-hydantoin (Methioninhydantoin) in einer alkalischen Prozesslösung, bei dem Nebenprodukte, umfassend Formiat und andere Anionen organischer Säuren, welche 1 bis 5 Kohlenstoffatome enthalten, mittels Elektrodialyse von der Prozesslösung abgetrennt werden.

## Beschreibung

Die vorliegende Erfindung betrifft ein Verfahren zur Herstellung von Methionin, umfassend die Hydrolyse von 5-(2-Methylmercaptoethyl)-hydantoin (Methioninhydantoin) in einer alkalischen Prozesslösung, bei dem Nebenprodukte, umfassend Formiat und andere Anionen organischer Säuren, welche 1 bis 5 Kohlenstoffatome enthalten, mittels Elektrodialyse von der Prozesslösung abgetrennt werden.

Die Aminosäure Methionin wird gegenwärtig weltweit in großen Mengen industriell hergestellt und ist von beträchtlicher kommerzieller Wichtigkeit. Methionin wird auf vielen Gebieten angewendet, wie zum Beispiel für pharmazeutische, Gesundheits- und Fitnessprodukte, insbesondere jedoch als Futtermitteladditiv in vielen Futtermitteln für verschiedene Nutztiere. Im industriellen Maßstab wird Methionin chemisch über die Bucherer-Bergs-Reaktion hergestellt, die eine Variante der Strecker-Synthese darstellt. Dabei werden die Ausgangssubstanzen 3-Methylmercaptopropanal (hergestellt aus 2-Propenal und Methylmercaptan), Blausäure (Cyanwasserstoff), Ammoniak und Kohlendioxid zum 5-(2-Methylmercaptoethyl)-hydantoin (Methioninhydantoin) umgesetzt und dieses anschließend alkalisch mit Alkalimetallhydroxid und/oder Alkalimetallcarbonat und Alkalimetallhydrogencarbonat, beispielsweise Kaliumhydroxid und/oder Kaliumcarbonat und Kaliumhydrogencarbonat, zum Alkalimethionat (z.B. Kaliummethioninat) hydrolysiert. Methionin wird schließlich durch Behandlung mit Kohlendioxid aus seinem Alkalimetallsalz, vorzugsweise seinem Kaliumsalz, freigesetzt, welches als Präzipitat aus der Alkalimetallcarbonat und Alkalimetallhydrogencarbonat (z.B. Kaliumcarbonat und Kaliumhydrogencarbonat) enthaltenden Mutterlauge abfiltriert werden kann (US 5,770,769). Die Reagenzien Ammoniak, Alkalimetallcarbonat und Alkalimetallhydrogencarbonat (z.B. Kaliumcarbonat und Kaliumhydrogencarbonat) sowie Kohlendioxid werden bei der industriellen Methioninproduktion in der Regel rezykliert. Mit der Zeit wird das in der Prozesslösung vorhandene Alkalimetallsalz (z.B. Kaliumsalz) in Form von neutralem Alkalimetallformiat (z.B. Kaliumformiat) inaktiviert. Alkalimetallformiat (z.B. Kaliumformiat) bildet sich aus den in der Methioninhydantoinlösung vorhandenen Resten an Blausäure und Alkalimetallsalzen aus der Hydantoinhydrolyse (WO2013030068A2). Weitere Nebenprodukte sind u.a. das Dipeptid Methionylmethionin ("Met-Met", EP1564208 A1), Acetat und 2-Hydroxy-4-methylmercaptobutansäure, das Hydroxy-Analogon des Methionins (MHA). Daher ist es erforderlich, kontinuierlich einen Teil der wässrigen Prozesslösung dieses Hydantoinhydrolysekreislaufs gegen frische wässrige Alkalimetallhydroxid-Lösung (z.B. Kaliumhydroxid-Lösung) auszutauschen. Generell muss die übermäßige Anreicherung von Nebenprodukten im Hydantoinhydrolysekreislauf vermieden werden, da ansonsten Störungen bei der Methionin-Kristallbildung auftreten.

Eine typische Prozesslösung im Hydantoinhydrolysekreislauf umfasst die folgenden Bestandteile: Ungefähr 10 bis 16 Gew. % Alkalimetall in Form von Alkalimetallsalzen, 5 bis 8 Gew. % Methionin, 3 bis 5 Gew. % Methionylmethionin, 0,7 bis 1,1 Gew. % Formiat und 0,2 bis 0,3 Gew.% Acetat. Eine typische Purge-Lösung umfasst die folgenden Bestandteile: Ungefähr 2 bis 6 Gew. % Methionin, 4 bis 8 Gew. % Methionylmethionin, 6 bis 14 Gew. % Alkalimetall in Form von Alkalimetallsalzen, 1 bis 1,7 Gew. % Formiat und 0,3 bis 0,5 Gew. % Acetat.

Da die ausgetauschte Prozesslösung (Purge) neben ca. 3 Gew. % inaktiviertem Alkalimetallsalz (z.B. Kaliumsalz) noch immer ca. 7-8 Gew. % aktives Alkalimetallsalz sowie Methionin als weiteren Wertstoff enthält, ist diese Verfahrensweise aber weder unter einer ökonomischen noch unter einer ökologischen Betrachtungsweise wünschenswert.

Eine bekannte Methode zur Aufbereitung von Abwässern oder zur Entsalzung von Wasser stellt die Elektrodialyse dar. Unter Elektrodialyse wird ein Prozess verstanden, bei welchem mittels Ionenaustauscher-Membranen und einer elektrischen Potenzialdifferenz ionogene Bestandteile aus einer wässrigen Lösung abgetrennt werden. Ein weiteres Einsatzgebiet der Elektrodialyse ist beispielsweise die Trennung von Aminosäuren, die unterschiedliche isoelektrische Punkte aufweisen, in Elektrodialysezellen, deren Kammern durch Anionen- und KationenaustauscherMembranen voneinander getrennt sind, bei verschiedenen pH-Werten unter dem Einfluss des elektrischen Feldes (H. Strathmann und H. Chmiel, Chem.-Ing.-Tech, 56 (1984) Nr. 3, 214-220).

Perry und Kedem (US 4,238,307 bzw. DE 29 07 450 A1) schlagen ein Elektrodialyseverfahren vor, mittels welchem sich in wässriger Lösung L-Aminosäuren von deren derivatisierten D-Enantiomeren, beispielsweise L-Methionin von N-Acetyl-D-Methionin, aufgrund deren unterschiedlichen isoelektrischen Punkte abtrennen lassen. Koberstein und Lehmann (DE 36 03 986 A1) offenbaren ein elektrodialytisches Verfahren zur Aufarbeitung der nach Abtrennung des Enzyms verbleibenden Lösung aus der Racematspaltung einer N-Acetyl-D,L-aminocarbonsäure in Gegenwart einer L-Aminosäurecyclase.

Bachot und Grosbois (US 4,454,012) offenbaren ein Verfahren zur Gewinnung von freiem, kristallinem Methionin aus dessen Alkalimetallsalz in wässriger, alkalimetallsalzhaltiger Prozesslösung unter Verwendung einer Elektrodialyseeinheit und unter Rückgewinnung von Alkalimetallhydroxid. Eine Methode zur Abtrennung von von Formiat, Acetat und anderen unerwünschten Anionen aus der Prozesslösung wird nicht offenbart.

Die CN105671587A offenbart ein Verfahren zur Herstellung von Methionin durch Verseifung von Methioninhydantoin, bei dem das entstandene Methioninalkalimetallsalz und Alkalimetallcarbonat durch Elektrodialyse in Methionin, Alkalimetallhydroxid und Kohlenstoffdioxid umgewandelt und getrennt werden. Bei diesem Verfahren wird Methioninhydantoin in einer Alkalimetallcarbonat enthaltenden Prozesslösung zum Methionin-Alkalimetallsalz hydrolysiert und durch wiederholte Behandlung in einem bipolare Membranen enthaltenden Elektrodialysesystem im schwach sauren Medium Methionin und Kohlenstoffdioxid freigesetzt und von der Prozesslösung abgetrennt. Durch Aufkonzentrieren und Kristallisieren wird Methionin erhalten. Alkalimetallcarbonat und Kohlenstoffdioxid werden zurückgewonnen. Methoden zur Abtrennung von Formiat, Acetat und anderen unerwünschten Anionen aus der Prozesslösung werden ebenfalls nicht offenbart.

Die CN106349131A offenbart ebenfalls ein Verfahren zur Herstellung von Methionin, bei dem das Methioninhydantoin mit Natriumhydroxid zum Methioninnatriumsalz verseift und das Methionin mit Schwefelsäure freigesetzt wird. Dabei wird durch Verwendung einer Elektrodialyseeinheit die Konzentration an Natriumsulfat in der wiederverwendeten Prozesslösung deutlich reduziert.

Aufgabe der vorliegenden Erfindung ist, ein Verfahren zur Herstellung von Methionin bereitzustellen, welches den Schritt der Hydrolyse von 5-(2-Methylmercaptoethyl)-hydantoin (Methioninhydantoin) in einer alkalischen Prozesslösung, die Freisetzung von Methionin aus dem gebildeten Alkalimetall-Methionat und die Rückführung der alkalischen Prozesslösung zum Hydrolyse-Schritt nach Abtrennung von Methionin umfasst, bei dem durch geeignete Maßnahmen die Konzentrationen an Nebenprodukten, umfassend Formiat und andere Anionen organischer Säuren soweit abgesenkt werden, dass die Menge an auszutauschender Prozesslösung (Purge) erniedrigt wird.

Die Aufgabe wird gelöst durch Verfahren zur Herstellung von Methionin, umfassend die alkalische Hydrolyse von 5-(2-Methylmercaptoethyl)-hydantoin (Methioninhydantoin) in einer Alkalimetallhydroxid und/oder Alkalimetallcarbonat und/oder Alkalimetallhydrogencarbonat enthaltenden wässrigen Prozesslösung zum Methioninalkalimetallsalz sowie die Ausfällung von Methionin durch Neutralisation mittels Kohlenstoffdioxid, Abtrennung des Methionins von der Prozesslösung, Einengung der Prozesslösung und Wiederverwendung dieser Prozesslösung für die alkalische Hydrolyse von der Prozesslösung erneut zugeführtem Methioninhydantoin, dadurch gekennzeichnet, dass Nebenprodukte, umfassend Formiat und andere Anionen organischer Säuren, welche 1 bis 5 Kohlenstoffatome enthalten, mittels Elektrodialyse von der Prozesslösung abgetrennt werden, wobei Methionin und/oder dessen Alkalimetallsalze im Wesentlichen in der Prozesslösung verbleiben und wobei jeweils eine Elektrodialysezelle mindestens zwei Kammern umfasst und eine dieser Kammern den Anodenraum darstellt, welcher durch eine ionenselektive Anionenaustauscher-Membran und eine Anode begrenzt wird, und im Anodenraum ein pH-Wert von 5,8 bis 8,0 vorliegt.

Dabei umfassen die Anionen organischer Säuren, welche 1 bis 5 Kohlenstoffatome enthalten, vorzugsweise Acetat und 2-Hydroxy-4-methylmercaptobutanoat (MHA).

Die Stromdichte in jeweils einer Elektrodialysezelle liegt vorzugsweise in einem Bereich von einschließlich 1,0 bis einschließlich 200 A/m².

Bei dem Verfahren gemäß der vorliegenden Erfindung umfasst jeweils eine Elektrodialysezelle drei Kammern, wobei eine Kammer den Kathodenraum darstellt, der durch eine Kationenaustauscher-Membran und eine Kathode begrenzt wird. Wahlweise kann jeweils eine Elektrodialysezelle auch nur zwei Kammern umfassen, wobei eine der beiden Kammern den Anodenraum und die andere Kammer den Kathodenraum darstellt, der durch die ionenselektive Anionenaustauscher-Membran und eine Anode begrenzt wird.

Bei dem Verfahren gemäß der vorliegenden Erfindung wird die Elektrodialyse bevorzugt in mindestens zwei Elektodialysezellen durchgeführt, welche jeweils mittels einer bipolaren Elektrode voneinander getrennt sind.

Bei einer besonderen Ausgestaltung des Verfahrens gemäß der vorliegenden Erfindung wird der pH-Wert im Anodenraum durch teilweise Zuführung der im Kathodenraum gebildeten Alkalimetalllauge eingestellt. Im Übrigen wird die im Kathodenraum gebildete Alkalimetalllauge der Prozesslösung wieder zugeführt.

Vorzugsweise ist das Alkalimetall in den Alkalimetallsalzen der Prozesslösung Kalium oder wahlweise Kalium und Natrium.

### Beispiele

### Versuchsaufbau

Die für die nachfolgenden Beispiele eingesetzte Elektrodialyszelle bestand aus drei Kammern. In der Anolytkammer wurde Kaliumhydrogencarbonat vorgelegt. In der Kathodenkammer wurde Kaliumhydroxid vorgelegt. In der mittleren Kammer wurde die Prozesslösung vorgelegt, welche unter andrem Methionin enthielt. Die Kreisläufe waren durch Ionenaustauschermembranen getrennt und wurden mit der jeweiligen Lösung durchströmt. Prozesslösungs- und Anolytkreislauf waren durch eine Anionenaustauschermembran getrennt, zwischen dem Prozesslösungs- und Katholytkreislauf befand sich eine Kationenaustauschermembran. Die Elektrolysezelle war an drei Kreislaufbehälter angeschlossen, die als Vorlage für die Lösungen dienten. Die Elektrodialysezelle wurde mit Gleichstrom von einem Netzgerät beaufschlagt. Im Anolytkreislauf wurde der pH-Wert kontrolliert, dieser durfte den Wert von pH 5,8 nicht unterschreiten und wurde durch Zudosierung von KOH konstant gehalten. Die Leitfähigkeit der 3 Lösungen wurde parallel aufgezeichnet.

### Technische Daten der verwendeten Elektrodialysezelle mit 3 Kammern:

| | |
|---|---|
| Anode: | Dimensionstabile Anode |
| Kathode: | Stahl |
| Anionenaustauschermembran: | Tokuyama Soda AMX |
| Kationenaustauschermembran: | Tokuyama Soda CMX |
| Elektrodenabstand: | 1,5 mm |
| Spacer: | PVC |
| Elektrodenfläche: | 200 cm² |

### Ausführung

In dem Salzkreislauf wurden 2 bzw. 5 kg (s. Beispiele) Prozesslösung vorgelegt. Im Katholyt wurden 2 kg einer 5 Gew.-% KOH und im Anolyt 2 kg einer 3 Gew.-% Kaliumformiatlösung vorgelegt.

Die Kreiselpumpen wurden eingeschaltet und der Druck in der Elektrodialyse durch Drosselung der Pumpen eingestellt. Die Druckdifferenz zwischen den Elektrodialysekammern wurde auf kleiner als 0,2 bar eingestellt. Die Durchflüsse waren in der Anolyt- und in der Katholyt-Kammer höher, da diese noch als Elektrodenspülung genutzt wurden. Der maximale Druck in den Kammern betrug 0,8 bar. Die Kreislaufgefäße des Anolyten und des Katholyten wurden mit Stickstoff überlagert. Das Netzgerät wurde eingeschaltet, so dass sich eine Stromdichte von 160 A/m² einstellte. Das Netzgerät wurde galvanostatisch betrieben.

### Beispiel 1

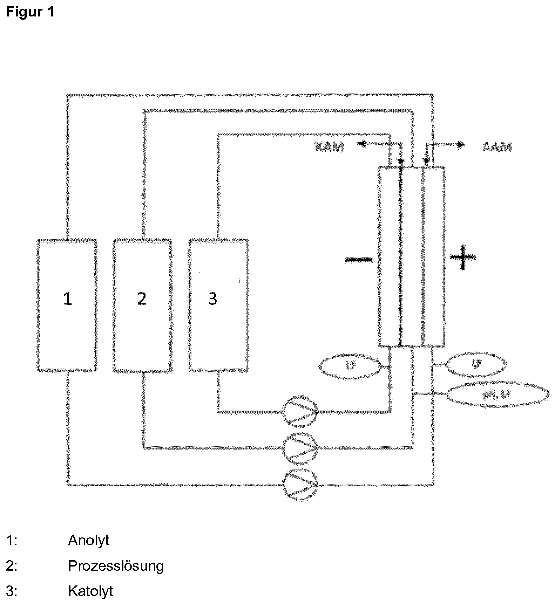

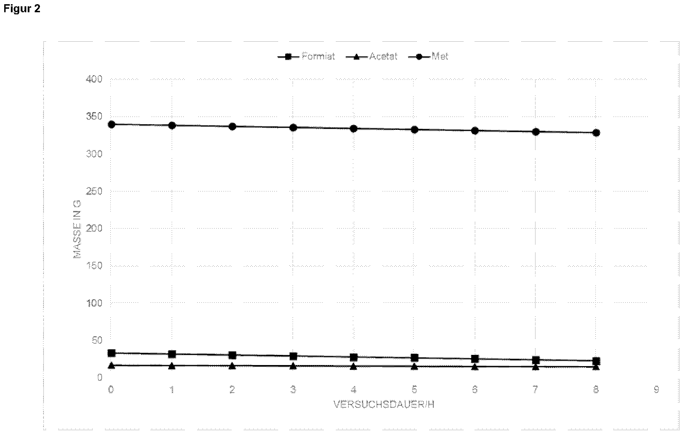

Es wurden 5 kg Prozesslösung mit der in Tabelle 1 angegebenen Zusammensetzung vorgelegt und 8 Stunden elektrodialysiert. Der pH Wert im Anolyt wurde durch Zugabe von KOH aus dem Katholyt auf pH 7 gehalten. Der Konzentrationsverlauf der einzelnen Komponenten während der Elektrodialyse ist in Tabelle 2 und in Figur 2 dargestellt.

### Beispiel 2

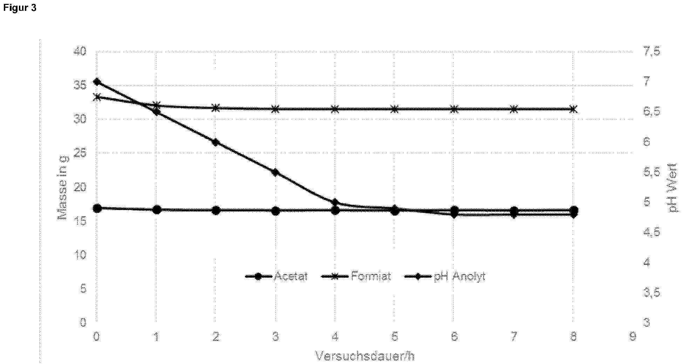

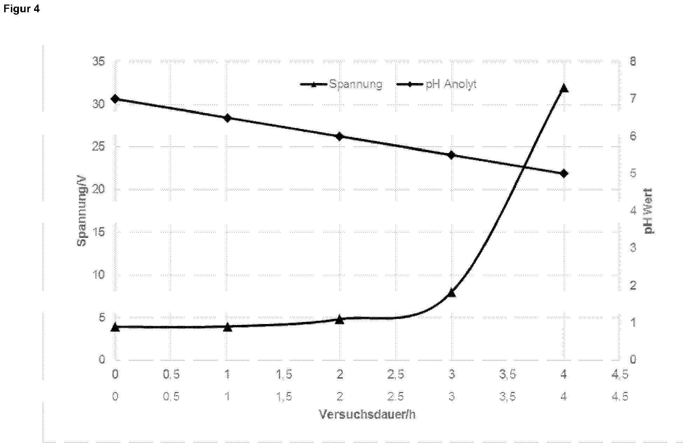

Es wurden 5 kg Prozesslösung mit der in Tabelle 3 angegebenen Zusammensetzung vorgelegt und 8 Stunden elektrodialysiert. Der pH Wert im Anolyt wurde nicht geregelt. Der Konzentrationsverlauf der einzelnen Komponenten und die Änderung des pH-Werts während der Elektrodialyse ist in Tabelle 4 und in Figur 3 dargestellt. Figur 4 zeigt den Verlauf des pH-Werts und der Spannung.

Wenn der pH im Anolyt unter 6 fällt, blockiert die Anionen-Austauschermembran. Die Spannung steigt schnell an, der Abbau bricht zusammen, da kein Strom mehr fließt (Figuren 3 und 4).

**Tabelle 1 (Beispiel 1)**

| **Ausgangszusammensetzung der 5 kg Prozesslösung in g** | | | | | |
|---|---|---|---|---|---|
| Formiat | Acetat | MHA | Met-Met | Kalium | Methionin |
| 33,35 | 16,97 | 29,73 | 10,71 | 630 | 340 |

**Tabelle 2 (Beispiel 1)**

| **Zeit** | **Absolute Mengen in der Prozesslösung in g** | | | | | |
|---|---|---|---|---|---|---|
| **h** | Formiat | Acetat | MHA | Met-Met | Kalium | Methionin |
| 0 | 33,35 | 16,97 | 29,72 | 10,71 | 630 | 340 |
| 1 | 32,07 | 16,71 | 29,70 | 10,63 | 627,15 | 338,59 |
| 2 | 30,79 | 16,46 | 29,67 | 10,55 | 624,31 | 337,18 |
| 3 | 29,51 | 16,20 | 29,64 | 10,48 | 621,46 | 335,77 |
| 4 | 28,23 | 15,95 | 29,61 | 10,40 | 618,62 | 334,37 |
| 5 | 26,96 | 15,70 | 29,58 | 10,32 | 615,77 | 332,96 |
| 6 | 25,68 | 15,44 | 29,56 | 10,24 | 612,93 | 331,55 |
| 7 | 24,40 | 15,19 | 29,53 | 10,16 | 610,08 | 330,14 |
| 8 | 23,12 | 14,94 | 29,50 | 10,08 | 607,24 | 328,73 |

**Tabelle 3 (Beispiel 2)**

| **Ausgangszusammensetzung der 5 kg Prozesslösung in g** | | | | | |
|---|---|---|---|---|---|
| Formiat | Acetat | MHA | Met-Met | Kalium | Methionin |
| 33,35 | 16,97 | 29,73 | 10,71 | 630 | 340 |

**Tabelle 4 (Beispiel 2)**

| **Zeit h** | **Absolute Mengen in der Prozesslösung in g** | | | | | | | |
|---|---|---|---|---|---|---|---|---|
| | | | | | | | **pH Anolyt** | **Spannung V** |
| | **Formiat** | **Acetat** | **MHA** | **Met-Met** | **Kalium** | **Methionin** | | |
| 0 | 33,35 | 16,97 | 29,73 | 10,7125 | 630 | 340 | 7 | 3,9 |
| 1 | 32,07 | 16,71 | 29,70 | 10,63 | 627,15 | 338,59 | 6,5 | 3,95 |
| 2 | 31,69 | 16,64 | 29,69 | 10,61 | 626,30 | 338,17 | 6 | 4,8 |
| 3 | 31,56 | 16,61 | 29,69 | 10,60 | 626,02 | 338,03 | 5,5 | 8,0 |
| 4 | 31,55 | 16,62 | 29,66 | 10,58 | 626,02 | 338,00 | 5 | 32,00 |
| 5 | 31,55 | 16,60 | 29,66 | 10,56 | 626,00 | 338,00 | 4,9 | 32,00 |
| 6 | 31,55 | 16,62 | 29,66 | 10,57 | 626,02 | 338,00 | 4,8 | 32,00 |
| 7 | 31,55 | 16,61 | 29,66 | 10,56 | 626,01 | 337,90 | 4,8 | 32,00 |
| 8 | 31,54 | 16,62 | 29,61 | 10,55 | 625,91 | 338,02 | 4,8 | 32,00 |

## Patentansprüche

1. Verfahren zur Herstellung von Methionin, umfassend die alkalische Hydrolyse von 5-(2-Methylmercaptoethyl)-hydantoin (Methioninhydantoin) in einer Alkalimetallhydroxid und/oder Alkalimetallcarbonat und/oder Alkalimetallhydrogencarbonat enthaltenden wässrigen Prozesslösung zum Methioninalkalimetallsalz sowie die Ausfällung von Methionin durch Neutralisation mittels Kohlenstoffdioxid, Abtrennung des Methionins von der Prozesslösung, Einengung der Prozesslösung und Wiederverwendung dieser Prozesslösung für die alkalische Hydrolyse von der Prozesslösung erneut zugeführtem Methioninhydantoin, **dadurch gekennzeichnet, dass** Nebenprodukte, umfassend Formiat und andere Anionen organischer Säuren, welche 1 bis 5 Kohlenstoffatome enthalten, mittels Elektrodialyse von der Prozesslösung abgetrennt werden, wobei Methionin und/oder dessen Alkalimetallsalze im Wesentlichen in der Prozesslösung verbleiben und wobei jeweils eine Elektrodialysezelle mindestens zwei Kammern umfasst und eine dieser Kammern den Anodenraum darstellt, welcher durch eine ionenselektive Anionenaustauscher-Membran und eine Anode begrenzt wird, und im Anodenraum ein pH-Wert von 5,8 bis 8,0 vorliegt

2. Verfahren nach Anspruch 1, wobei die Anionen organischer Säuren, welche 1 bis 5 Kohlenstoffatome enthalten, Acetat und 2-Hydroxy-4-methylmercaptobutanoat (MHA) umfassen.

3. Verfahren nach einem der Ansprüche 1 oder 2, bei dem die Stromdichte in jeweils einer Elektrodialysezelle in einem Bereich von einschließlich 1,0 bis einschließlich 200 A/m² liegt.

4. Verfahren nach einem der Ansprüche 1 bis 3, bei dem jeweils eine Elektrodialysezelle drei Kammern umfasst, wobei eine Kammer den Kathodenraum darstellt, der durch eine Kationenaustauscher-Membran und eine Kathode begrenzt wird.

5. Verfahren nach einem der Ansprüche 1 bis 3, bei dem jeweils eine Elektrodialysezelle zwei Kammern umfasst und eine der beiden Kammern den Anodenraum und die andere Kammer den Kathodenraum darstellt, der durch die ionenselektive Anionenaustauscher-Membran und eine Kathode begrenzt wird.

6. Verfahren nach einem der Ansprüche 1 bis 5, wobei die Elektrodialyse in mindestens zwei Elektodialysezellen durchgeführt wird, welche jeweils mittels einer bipolaren Elektrode voneinander getrennt sind.

7. Verfahren nach einem der Ansprüche 1 bis 6, wobei der pH-Wert im Anodenraum durch teilweise Zuführung der im Kathodenraum gebildeten Alkalimetalllauge eingestellt wird.

8. Verfahren nach einem der Ansprüche 1 bis 6, wobei die im Kathodenraum gebildete Alkalimetalllauge der Prozesslösung wieder zugeführt wird.

9. Verfahren nach einem der Ansprüche 1 bis 8, bei dem das Alkalimetall Kalium ist.

10. Verfahren nach einem der Ansprüche 1 bis 8, bei dem das Alkalimetall Kalium und Natrium ist.
